# EUROPEAN PATENT APPLICATION

(11) **EP 1 762 849 A1**
(43) Date of publication of application: **14.03.2007**
(21) Application number: 06254699.9
(22) Date of filing: 08.09.2006
(51) Int. Cl.: G01N 33/50, A61K 9/00

(54) **Method for demonstrating efficacy of a topically applied active ingredient**

(30) Priority: 09.09.2005 US 222887
(71) Applicant: Johnson and Johnson Consumer Companies, Inc., Skillman, NJ 08558 (US)
(72) Inventor: Garay, Michelle, Pittstown, NJ 08867 (US); Southall, Michael, Lawrenceville, NJ 08558 (US)
(74) Representative: James, Anthony Christopher W.P.

(57) **Abstract**

A method is disclosed for demonstrating the efficacy of a topically applied active ingredient, which method includes topically applying a composition containing an active ingredient to an area of skin, applying an absorbent material to the area of skin to extract at least a portion of the active ingredient from the skin, thereby incorporating the portion of active ingredient with the absorbent material in a releasable manner, removing the absorbent material from the skin, extracting the active ingredient from the absorbent material, combining the extracted active ingredient with cellular growth media, incubating the cellular growth media with cultured cells for a period of time to produce a biomarker, and measuring the biomarker to indicate efficacy of the active ingredient.

## Description

### Field of the Invention

The present invention relates to a method for demonstrating efficacy of a topically applied active ingredient. The method is useful for supporting time-based efficacy claims.

### Background of the Invention

The consumer market is replete with creams, lotions, sprays and the like that are intended for providing skin care benefits. The benefits targeted include moisturization, wrinkle reduction, skin smoothing, acne treatment, reduced inflammation, and depigmentation. Many of these benefits are delivered through topical application of a composition containing an active ingredient.

Test methods have been developed to demonstrate the efficacy of topically applied active ingredients. These test methods typically analyze for the presence of the active ingredient, and then efficacy is inferred from the mere presence of the active. For example, United States Patent Application Number 2002/ 0019055 discloses a non-invasive test for substances on the surface of the skin. An absorbent pad is placed on the skin for a defined time period, removed, and then analyzed for the substance of interest. An analytical method can also be incorporated as part of the absorbent pad in order to conduct both the extraction of the chemical of interest and the assay in situ. Although this method quantifies the analyte, it does not demonstrate the efficacy of the analyte. Therefore, there is a need for a test method that demonstrates the efficacy of a topically applied active ingredient.

### Summary of the Invention

The present invention provides a method for demonstrating the efficacy of a topically applied active ingredient including: topically applying a composition containing a therapeutically effective amount of an active ingredient to an area of skin; applying an absorbent material to the area of skin for a time effective to extract at least a portion of the active ingredient from the skin, thereby incorporating the portion of active ingredient with the absorbent material in a releasable manner; removing the absorbent material comprising the incorporated active ingredient from the area of skin; extracting the incorporated active ingredient from the absorbent material, combining the extracted active ingredient with a cellular growth media containing cultured cells; incubating the cellular growth media with cultured cells for a period of time effective to produce a biomarker in the growth media; and measuring the biomarker to indicate efficacy of the active ingredient.

### Detailed Description of the Invention

The first step in the method of the present invention is topically applying a composition containing an active ingredient to an area of the skin. As used herein, topically applying means applying a composition to the surface of the skin by hand, spray, mist, applicator or any means known in the art. Suitable compositions include powders, solutions, creams, lotions, ointments and the like. The compositions contain at least one active ingredient in a therapeutically effective amount. As used herein, therapeutically effective amount is that amount required to provide the therapeutic benefit sought by application of the composition to the skin area. Suitable active ingredients include those intended to provide a skin benefit selected from moisturization, anti-aging, skin smoothing, acne treatment, reduced inflammation, itch, depigmentation, and combinations thereof. Suitable active ingredients include, but are not limited to, retinol, dimethylaminoethanol, tetrahydroxypropyl ethylenediamine, soy, feverfew, hydrocortisone, salicylic acid, benzoyl peroxide and the like. The composition is left on the skin for a period of time sufficient to provide the skin benefit being sought, or for the pre-determined time at which efficacy is desired to be proven. For example, the composition may be present on the skin for about 1 to about 12 hours, or up to 24 hours, prior to proceeding with the next step. The data may be used for regulatory or marketing purposes.

The second step in the method of the present invention is applying an absorbent material to the area of skin to which the active ingredient has been applied for a time effective to extract at least a portion of the active ingredient from the skin for further efficacy analysis. As used herein, absorbent material includes those materials into which the active ingredient is absorbed or to which the active ingredient may be otherwise adhered or bound. The absorbent material utilized is not critical, so long as the active ingredient is capable of being releasably bound to or otherwise releasably incorporated with the absorbent material. The active may be absorbed by or adhered to the absorbent material, either by chemical or mechanical binding. Examples of suitable absorbent materials include, but are not limited to, adhesives, powders, films, swabs, and wipes

The absorbent material may contain solvents to dissolve and extract the active ingredient from the skin. The solvent may be organic, aqueous, or mixtures thereof. Preferably, an adhesive is utilized to remove the active ingredient from the skin. Suitable adhesives include aqueous-based adhesives, solvent-based adhesives and hot melt adhesives. Examples of suitable adhesives include, but are not limited to, those based on styrenic block copolymers and tackifying resins such as HL-1491 available from HB-Fuller Co. (St. Paul MN), H-2543 available from ATO-Findley (Wawatausa, WI), and Resyn 34-5534 available from National Starch & Chemical Company (Bridgewater, NJ). Ethylene copolymers, including ethylene vinyl acetate copolymers, are also useful as adhesives.

Suitable adhesives also include acrylic-based, dextrin-based, and urethane-based adhesives, as well as natural and synthetic elastomers. The adhesives may also include amorphous polyolefins including amorphous polypropylene, such as HL-1308 available from HB Fuller or Rextac RT 2373 available from Huntsman (Odesssa, TX). The adhesive may be based on Kraton® Brand synthetic elastomers, or natural rubber. These adhesives may also include tackifiers, anti-oxidants, processing oils, and the like as is known in the art.

In one embodiment, the adhesive may be coated on a substrate such as paper or a polymeric film, such as a tape, and then applied to the skin. The tape is left on the skin for a sufficient time to extract the active ingredient from the skin. The amount of time that the tape is left on the skin will depend on the adhesive and the active ingredient, but typically may range from 1 second to 1 hour.

The third step of the present invention is removing the absorbent material comprising the portion of active ingredient releasably incorporated therewith from the skin. The absorbent material is typically peeled off of the skin in manners known in the art.

The fourth step of the present invention is extracting all or a part of the portion of active ingredient releasably incorporated with the absorbent material from the absorbent material and combining with cellular growth media for analysis. The active ingredient may be extracted using solvents that are suitable for extraction of the particular active ingredient being extracted. Those skilled in the art will be able to readily ascertain the appropriate solvent for the particular active ingredient once having the benefit of this disclosure. Selected solvents also must be non-cytotoxic to cells used in methods of the present invention. In certain embodiments, the absorbent material may be placed in cellular growth media and the active ingredient extracted in the presence of the growth media. Any growth media supportive of living cells may be utilized. One such growth media is RPMI Medium 1640.

The fifth step of the present invention is incubating cultured cells in the cellular growth media for a period of time effective to produce a biomarker in order to determine efficacy of the active ingredient at the time of removal from the skin. The living cells may be of various cell types. Suitable examples of living cells that are useful in the process of the present invention include, but are not limited to, murine cells, and human-derived cells such as Hacat keratinocyte cells and Jurkat T-cells. The period for incubation may vary, depending on the biomarker being measured, but typically is anywhere from 1 minute to 24 hours, for example 8 hours or 16 hours.

The last step of the present invention is analyzing the cultured cells for specific biomarkers indicative of the efficacy desired Suitable biomarkers include, but are not limited to, cytokines, for example interleukin 1, interleukin 2 ("IL-2"), or interleukin 6; and reactive oxygen species, such as alcohols and peroxides. The biomarkers produced by the cells may be quantified by methods known in the art, such as high performance liquid chromatography, gas chromatography, enzyme linked immunoassays and the like.

Several examples are set forth below to further illustrate the nature of the invention and the manner of carrying it out. However, the invention should not be considered as being limited to the details thereof.

### Examples

i. One hour following topical application of ~4mg/cm² of *Clean & Clear Persa-Gel* ®*10,* a 10% benzoyl peroxide topical cream, a Sebutape was applied to this skin area. Hacat (keratinocyte) cells were treated with the extracted material from the tape strips, and subsequently assayed for reactive oxygen species ("ROS") with detection of hydrogen peroxide as an endpoint. Cells were also treated with a 1:1000 dilution of the *Persa-Gel* ointment, and a 1:1000 dilution of 30% hydrogen peroxide. In comparison to untreated cells (125 M.F.U. H₂O₂), detection of the peroxide species from the extracted tape strips (400 M.F.U. H₂O₂) was comparable to the 1:1000 treatment with the ointment (350 M.F.U. H₂O₂). Therefore, this methodology detected the presence and activity *in vitro* of a topically applied anti-acne medication on the skin following extraction from skin tapes.
ii. Three panelists were selected for a test to demonstrate the efficacy of hydrocortisone creams over time. Four rectangles were marked on the inner volar forearm of each panelist, with placement of the outer edge of the 1^{st} square beginning approximately one-half inch from the elbow crease. Markings were then placed on the skin with non-smearable ink on the inside corners of the rectangular box. Products containing 1% hydrocortisone were applied in a uniform thin line in the approximate center on the rectangle, beginning from the top to the bottom (32 ul/rectangle). Each product was then carefully rubbed into the rectangle area only, with a circular motion, for approximately 10 seconds.

After the indicated time following product application, Sebutape® strips were applied to the appropriate skin locations. With gloved hands, the strips were removed from the sheet with forceps and applied to the center of the skin rectangle, pressed firmly, and removed 1 minute later with forceps. The tapes were then placed skin-side-down in appropriately labeled vials. Finally, 500µl of cell growth media RPMI 1640 was added to each vial and the vials placed in a -80°C freezer until time of IL-2 assay

The vials were thawed on ice, and then sonicated on ice for 15 minutes. Jurkat cells were plated onto 96-well round bottom plates at 100,000 cells/well in 100µl. Cells were then stimulated for IL-2 production with the addition of 50µl mixture of phorbol myristate acetate ("PMA", 200ng/ml) and phytohemagglutinin ("PHA", 16µg/ml). Designated sample wells were then treated with 50µl of media from sample vials after vortexing them for 10 seconds. Each sample was used to treat 2 wells. To the wells designated for stimulation only, 50µl of RPMI cellular growth media + 10% fetal bovine serum ("FBS") growth media was added. Plates were incubated overnight for approximately 16 hours @ 37°C and 5% CO₂.

After incubation, the supernatants were removed and transferred to low-binding 96-well plates. The supernatants were diluted 1:5 in RMPI growth media and assayed for IL-2 concentration using the Upstate kit according to the manufacturers protocol and analyzed on a Luminex 100 multi-analyte detector (Luminex Corp, Austin, TX). Values from the Luminex were correlated to actual IL-2 concentration values using a standard curve from known IL-2 concentrations included on the plate. The average concentration in the stimulated wells was determined as the normal IL-2 release.

The calculated concentrations in treated wells were used to calculate a percent inhibition of this normal value. Each plate contained a set of stimulated wells and this calculation was made separately for each plate. Panelist results were compiled to compare results of each product at each time point. Unpaired Student's T-tests were performed to evaluate the significance of differences between groups with significance levels in all tests set at values <0.05.

The results demonstrated that hydrocortisone treated skin inhibited IL-2 production at 1 hour, 7 hours, and 12 hours. The average percent IL-2 inhibition was 66 at 1 hour, 61 at 7 hours, and 67 at 12 hours. This data supports the efficacy of the hydrocortisone creams tested over these time periods.

## Claims

1. A method for demonstrating the efficacy of a topically applied active ingredient, comprising:
topically applying a composition containing an active ingredient to an area of skin,
applying an absorbent material to the area of skin to remove at least a portion of the active ingredient from the area of skin, thereby incorporating the portion of active ingredient with the absorbent material in a releasable manner,
removing the absorbent material comprising the portion of active ingredient incorporated therewith from the skin,
extracting the active ingredient from the absorbent material,
combining the extracted active ingredient with a cellular growth media comprising cultured cells,
incubating cultured cells in the cellular growth media for a period of time effective to produce a biomarker; and
measuring the biomarker to indicate efficacy of the active ingredient.

2. The method according to Claim 1 wherein the active ingredient is selected from the group consisting of retinol, dimethylaminoethanol, tetrahydroxypropyl ethylenediamine, soy, feverfew, hydrocortisone, salicylic acid and benzoyl peroxide.

3. The method according to Claim 1 wherein the absorbent material is selected from the group consisting of adhesives, swabs, powders, films and wipes.

4. The method according to Claim 1 wherein the cellular growth media is RPMI Medium 1640.

5. The method according to Claim 1 wherein the cells are selected from the group consisting of murine cells, Hacat keratinocyte cells and Jurkat T-cells.

6. The method according to Claim 1 wherein the biomarker is selected from the group consisting of interleukin 1, interleukin 2, interleukin 6 and reactive oxygen species.

7. The method according to Claim 1 wherein the absorbent material is applied to the area of skin 1 to 24 hour after applying the composition comprising the active ingredient to the area of skin.

8. The method according Claim 1 wherein the portion of active ingredient is extracted from the absorbent material in the presence of the cellular growth media.

9. The method according to Claim 1 wherein the cells are incubated in the cellular growth media for about 1 minute to about 24 hours.
